# EUROPÄISCHE PATENTSCHRIFT

(11) **EP 2 234 536 B1**
(45) Veröffentlichungstag und Bekanntmachung des Hinweises auf die Patenterteilung: **25.04.2018**
(21) Anmeldenummer: 09705764.0
(22) Anmeldetag: 21.01.2009
(51) Int. Cl.: A61B 5/00, A61B 5/151, G01N 35/00, G01N 35/10

(54) **SYSTEM ZUM NACHWEIS EINES ANALYTEN IN EINER KÖRPERFLÜSSIGKEIT**
SYSTEM FOR DETECTION OF AN ANALYTE IN A BODY FLUID
SYSTÈME DE DÉTECTION D'UN ANALYTE DANS UN LIQUIDE CORPOREL

(30) Priorität: 28.01.2008 US 20766
(43) Veröffentlichungstag der Anmeldung: 06.10.2010
(73) Patentinhaber: Roche Diabetes Care GmbH, 68305 Mannheim (DE); F. Hoffmann-La Roche AG, 4070 Basel (CH)
(72) Erfinder: WONG, Daniel, Sunnyvale California 94087 (US); PATEL, Paul, Sunnyvale California 94086 (US); PETRICH, Wolfgang, 76669 Bad Schoenbron (DE); VRANCIC, Christian, 68165 Mannheim (DE)
(74) Vertreter: Herzog, Fiesser & Partner Patentanwälte PartG mbB
(86) Internationale Anmeldenummer: PCT/EP2009/050640
(87) Internationale Veröffentlichungsnummer: WO 2009/095343

(56) Entgegenhaltungen:
- EP-A- 0 988 828
- EP-A- 1 881 322
- WO-A-01/72220
- WO-A-2007/111651
- WO-A1-2004/086970
- WO-A1-2007/045412
- US-A1- 2003 083 686
- US-A1- 2007 016 103
- US-B1- 6 591 125

## Beschreibung

### Gebiet der Erfindung

Die Erfindung betrifft ein System zum Nachweis mindestens eines Analyten in einer Körperflüssigkeit. Derartige Systeme werden beispielsweise als tragbare Nachweisvorrichtungen oder auch in stationären Vorrichtungen eingesetzt, um insbesondere einen oder mehrere Analyten in Körperflüssigkeiten wie Blut oder interstitieller Flüssigkeit qualitativ oder quantitativ nachzuweisen. Als Analyten kommen insbesondere Metaboliten in Betracht. Im Folgenden wird, ohne Beschränkung weiterer Arten von Analyten, insbesondere der Nachweis von Blutglucose beschrieben.

### Stand der Technik

Aus dem Stand der Technik sind zahlreiche Systeme zum Nachweis von Analyten in Körperflüssigkeiten bekannt. Diese Systeme basieren in der Regel darauf, dass zunächst eine Probe der Körperflüssigkeit generiert wird, beispielsweise durch Verwendung mindestens einer Lanzette. Anschließend wird diese Probe in der Regel unter Verwendung mindestens eines Testelements qualitativ oder quantitativ auf den mindestens einen nachzuweisenden Analyten hin untersucht. Dies kann beispielsweise auf optischem und/oder elektrochemischem Wege erfolgen. Beispielsweise kann das Testelement ein oder mehrere Testfelder enthalten, mit einer Testchemie, welche speziell für den Nachweis des mindestens einen Analyten ausgestaltet ist. Beispielsweise kann die Testchemie bei Anwesenheit des mindestens einen Analyten eine oder mehrere nachweisbare Reaktionen durchführen oder Veränderungen ausgesetzt sein, welche beispielsweise auf physikalischem und/oder chemischem Wege nachgewiesen werden können.

Aus dem Stand der Technik sind zahlreiche derartige Systeme bekannt. So beschreibt beispielsweise US 7,252,804 B2 eine Messeinheit zum Analysieren einer Körperflüssigkeit, umfassend ein auf der Verwendung von Teststreifen basierendes Messgerät sowie eine mit dem Messgerät verbundene Lanzette. Weiterhin sind auch Systeme bekannt, bei welchen eine Probengenerierung und eine Aufnahme der Probe durch ein Testelement kombiniert werden. Beispielsweise beschreibt EP 1 992 283 A1 ein Stechsystem mit Lanzetten zum Erzeugen einer Stichwunde sowie Probenaufnahmeeinrichtungen zum Aufnehmen einer Körperflüssigkeitsprobe. Anschließend an eine Stechbewegung wird eine Probenaufnahmebewegung durchgeführt, bei welcher die Probe aufgenommen wird. In ähnlicher Weise beschreibt EP 1 881 322 A1 ein tragbares Messsystem zur Analyse einer flüssigen Probe, welches ein feuchtigkeitsdichtes Gehäuse mit einer Gehäuseinnenatmosphäre aufweist. Dabei ist die flüssige Probe innerhalb der Gehäuseinnenatmosphäre auf das mindestens eine Testelement aufbringbar.

Neben derartigen Systemen, bei welchen eine Probe generiert und anschließend auf das Testelement übertragen wird, existieren Systeme, bei denen eine integrierte Probenerzeugung und Probenaufnahme erfolgt. Beispielsweise kann dies mittels entsprechender Nadeln erfolgen, welche ganz oder teilweise als Kapillaren zur Aufnahme der Flüssigkeitsprobe ausgestaltet sind. Mittels dieser Kapillaren kann die Flüssigkeitsprobe auf ein Testelement übertragen werden, welches beispielsweise in die Nadel oder allgemein in eine Lanzettenvorrichtung integriert sein kann. Derartige Lanzettensysteme werden häufig auch als "Get and Measure"-Systeme bezeichnet. Beispiele derartiger integrierter Lanzettensysteme sind in WO 2005/084546 A2 beschrieben.

Unabhängig vom verwendeten System besteht bei Systemen zum Nachweis von Analyten in Körperflüssigkeiten allgemein die Bestrebung, ein Probenvolumen der Proben erheblich zu reduzieren. Eine derartige Reduktion ist aus mehreren Gründen wünschenswert. Zum einen lässt sich durch verringerte Probenvolumina der Schmerz, welcher für den Patienten mit der Analyse verbunden ist, vermindern. Weiterhin bereiten größere Probenvolumina auch Schwierigkeiten, beispielsweise bezüglich einer erhöhten Gefahr einer Kontamination der Analysegeräte durch die Probe selbst. Ein weiterer Grund für die Reduzierung von Probenvolumina besteht in der Bestrebung, integrierte Systeme herzustellen. Diese Integration bedingt eine höhere Funktionalität bei gleichem Bauraum, so dass in der Regel der Bauraum für eine Lanzette reduziert ist und damit auch das Probenvolumen. Weiterhin entfällt bei diesen Systemen in der Regel die Möglichkeit einer aktiven Manipulation der perforierten Hautoberfläche zur Erhöhung des Probenvolumens ("Melken"), so dass integrierte Systeme zumeist mit geringeren Probenvolumina arbeiten müssen.

Eine Schwierigkeit bei Systemen, welche mit verminderten Probenvolumina arbeiten, beispielsweise Blutvolumina unterhalb 1 µl, kann jedoch, wie im Rahmen der vorliegenden Erfindung herausgefunden wurde, in einem Einfluss der Verdunstung und einer damit verbundenen zumindest teilweisen Eintrocknung der Probe liegen. Eine Eintrocknung der Probe, beispielsweise durch Verdunstung von Wasser, bewirkt jedoch wiederum eine Aufkonzentrierung der in der flüssigen Probe gelösten Substanzen, wie beispielsweise Glucose. Bei derartig aufkonzentrierten Proben werden dann jedoch fälschlicherweise erhöhte Konzentrationen gemessen.

Verdunstungseffekte von Flüssigkeiten allgemein sind vielfach untersucht und in zahlreichen Veröffentlichungen in der Literatur beschrieben. Die meisten Untersuchungen beziehen sich dabei auf frei fallende Wassertropfen oder aufgesetzte Wassertropfen, nicht jedoch auf Flüssigkeiten allgemein in Vertiefungen, welche sich fundamental unterschiedlich zu freien Tropfen verhalten können. Die Verdunstung wird beispielsweise durch die Luftfeuchtigkeit und die Konvektion in der Umgebung der Flüssigkeitsoberfläche beeinflusst. Typische Verdunstungsraten von Tropfen von zumindest näherungsweise 100 nl bewegen sich bei Normalbedingungen im Bereich von 0,3 bis 0,6 nl/s und sind bei konstanten Umgebungsbedingungen durch beispielsweise die Tropfenoberfläche bedingt.

Die genannten Grundlagenuntersuchungen führen in vielen Fällen zu komplexen theoretischen Vorhersagen einer Verdunstung, welche auf der Kenntnis einer Vielzahl von Umgebungsbedingungen und Parametern basieren. Da Analysesysteme für den Nachweis von Analyten in Körperflüssigkeiten jedoch in vielen Fällen über einen weiten Temperatur- und Luftfeuchtigkeitsbereich und unabhängig von speziellen Konvektionsbedingungen arbeiten müssen, sind derartige Vorhersagen und Analysen jedoch in der Praxis vergleichsweise wenig hilfreich.

Auch aus dem Bereich der medizinischen Diagnostik sind Einflüsse von Trocknungseffekten bekannt. In US 7,252,804 B2 wird beispielsweise auf den Effekt einer derartigen Eintrocknung von Blutproben bei Biosensoren mit Stechhilfen hingewiesen. In analoger Weise wird in US 6,878,262 B2 dieser Effekt vermerkt und vorgeschlagen, Kapillaren für den Bluttransport abzuschließen, um Verdunstungen zu vermeiden. Eine analoge Vorgehensweise wird auch beispielsweise in US 6,565,738 B1 oder in US 6,312,888 B1 gewählt. Auch in US 6,325,980 B1 wird zur Vermeidung einer Austrocknung von Proben, insbesondere durch Konvektion, eine Abdeckung der Proben mit einem Volumen von weniger als 0,5 µl vorgeschlagen.

Viele der bekannten Ansätze begegnen also dem Verdunstungsproblem durch Abdeckung der Kapillaren, was jedoch in der Praxis in vielen Fällen kaum oder nur schlecht realisierbar ist. Insbesondere bei den oben beschriebenen "Get and Measure"-Systemen ist eine Abdeckung der als Einweg-Systeme konstruierten Lanzetten technisch nur unter vergleichsweise hohem Aufwand realisierbar. In vielen Fällen ist daher die Verdunstung aus halboffenen Kapillaren zu betrachten. Derartige Systeme mit einer Mehrzahl von Grenzflächen sind jedoch theoretisch nur schwer zu beschreiben. Aufgrund der oben genannten komplexen Umgebungsbedingungen, insbesondere hinsichtlich des Temperatur- und/oder Luftfeuchtigkeitsbereichs und der speziellen Konvektionsbedingungen, ist es insbesondere nicht ausreichend, konstante Korrekturfaktoren in die Berechnung einer Glucosekonzentration und/oder einer anderen Analytkonzentration einzubringen. In der Praxis hat es sich insbesondere gezeigt, dass theoretische oder semi-empirische Ansätze zur Korrektur der Verdunstung in vielen Fällen zu unrealistisch niedrigen Verdunstungsraten und damit fehlerhaften Korrekturen führen. Ferner wird auf die Dokumente US6591125 B1 und WO2004/086970 A1 verwiesen.

### Aufgabe der Erfindung

Es ist daher eine Aufgabe der vorliegenden Erfindung, ein System zum Nachweis mindestens eines Analyten in einer Körperflüssigkeit bereitzustellen, welches die Nachteile bekannter Systeme vermeidet. Das System soll insbesondere kostengünstig herstellbar sein und soll dennoch in der Lage sein, unter einer weiten Bandbreite realistischer Umgebungsbedingungen verbesserte Nachweisergebnisse zu erbringen.

### Offenbarung der Erfindung

Es wird daher ein System zum Nachweis eines Analyten in einer Körperflüssigkeit mit den Merkmalen der unabhängigen Ansprüche vorgeschlagen. Vorteilhafte Weiterbildungen der Erfindung, welche einzeln oder in Kombination realisiert werden können, sind in den abhängigen Ansprüchen dargestellt.

Das System dient dem Nachweis eines Analyten in einer Körperflüssigkeit. Bei dieser Körperflüssigkeit kann es sich insbesondere um Blut und/oder interstitielle Flüssigkeit handeln, wobei jedoch alternativ oder zusätzlich auch andere Arten von Körperflüssigkeit untersucht werden können. Bei dem mindestens einen Analyten, welcher qualitativ und/oder quantitativ nachgewiesen werden kann, kann es sich insbesondere um mindestens einen Metaboliten handeln. Insbesondere kann es sich um Blutglucose handeln. Alternativ oder zusätzlich können jedoch auch Analyten wie beispielsweise Cholesterin, Laktat, Coagulate, Troponin, Myoglobin, proBNP, C-reaktives Protein, CK-MB oder Ähnliches nachgewiesen werden. Auch ein Nachweis einer Kombination mehrerer Analyten ist möglich.

Das vorgeschlagene System ist eingerichtet, um eine Probe der Körperflüssigkeit zu generieren und auf mindestens ein Testelement, insbesondere ein Testfeld, zu transferieren. Zum Zweck der Generierung der Probe kann das System insbesondere mindestens eine Lanzette zum Perforieren einer Hautpartie eines Benutzers umfassen. Der Begriff einer Lanzette ist dabei weit zu fassen und umfasst grundsätzlich ein beliebiges Element, welches einen Einschnitt und/oder Einstich in die Hautpartie generieren kann. Weiterhin können die Lanzette und/oder das System mindestens einen Aktor umfassen, welcher eingerichtet ist, um eine Lanzettenbewegung des Elements zur Generierung des Einschnitts und/oder Einstichs zum Zweck einer Generierung der Probe der Körperflüssigkeit zu realisieren.

Das mindestens eine Testelement kann beispielsweise mindestens ein Testfeld umfassen. Insbesondere kann das mindestens eine Testelement, insbesondere das mindestens eine Testfeld, mindestens eine Testchemie umfassen, welche bei Anwesenheit des mindestens einen Analyten mindestens eine messbare Eigenschaft, beispielsweise eine physikalisch und/oder chemisch messbare Eigenschaft, ändert. Beispielsweise kann es sich dabei um eine elektrochemisch messbare und/oder optisch messbare Eigenschaft handeln, beispielsweise eine Farbänderung. Zu diesem Zweck kann das Testelement, insbesondere die Testchemie, entsprechende Chemikalien und/oder Chemikalienmischungen, beispielsweise Enzyme, Hilfsstoffe oder Ähnliches, umfassen, welche aus dem Stand der Technik grundsätzlich bekannt sind und auch im Rahmen der vorliegenden Erfindung eingesetzt werden können. Beispielsweise kann auf J. Hönes et al., Diabetes Technology and Therapeutics, Volume 10, Supplement 1, 2008, S.10 - S.26, verwiesen werden. Die dort beschriebenen Testelemente und/oder Testchemikalien sind auch im Rahmen der vorliegenden Erfindung einsetzbar.

Das Testelement kann in die mindestens eine Lanzette integriert sein und/oder kann von der mindestens einen Lanzette auch ganz oder teilweise getrennt ausgebildet sein. Ist das Testelement in die mindestens eine Lanzette integriert, so können beispielsweise aus bekannten "Get and Measure"-Systemen bekannte Lanzetten eingesetzt werden, beispielsweise gemäß dem oben zitierten Stand der Technik. Beispielsweise können ein oder mehrere Testfelder am Ende einer geschlossenen oder geöffneten Kapillare angeordnet sein oder auch einen Kapillarspalt ganz oder teilweise überdecken, so dass die Probe durch den Kapillarspalt auf das Testelement übertragen wird.

Um den Transfer der Probe, welcher vollständig oder teilweise erfolgen kann, auf das mindestens eine Testelement zu gewährleisten, können mehrere Mechanismen vorgesehen sein. So kann beispielsweise der Transfer von dem Ort der Generierung zu dem Testelement zumindest teilweise beziehungsweise abschnittsweise über mindestens eine Kapillare erfolgen. Insbesondere kann es sich dabei um eine Kapillare handeln, welche ganz oder teilweise in eine Lanzette integriert ist. Insbesondere kann diese Kapillare als teilweise geöffnete Kapillare, also als Kapillare in Form eines geöffneten Schlitzes in der Lanzette, ausgestaltet sein. Insbesondere kann es sich dabei, wie oben beschrieben, um eine Lanzette mit einem integrierten Testelement handeln, also eine so genannte "Get and Measure"-Lanzette.

Der Begriff des Transfers der Probe der Körperflüssigkeit auf das Testelement ist allgemein jedoch weit zu fassen. Dieser Begriff bedeutet allgemein, dass die Probe und das Testelement relativ zueinander bewegt werden sollen, das heißt in ihrer Position und/oder Orientierung und/oder Ausdehnung und/oder Form geändert werden sollen, so dass die Probe auf das Testelement übertragen wird. Die Verwendung einer Kapillare ist eine Möglichkeit, um die Probe selbst zu dem Testelement zu bewegen. Alternativ oder zusätzlich kommen jedoch auch Mechanismen in Betracht, bei welchen das Testelement bewegt wird, um die Probe aufzunehmen. So kann beispielsweise in dem System ein Mechanismus vorgesehen sein, bei welchem zunächst die Probe auf und/oder in einer Hautpartie generiert wird und anschließend durch eine relative Bewegung des Testelements relativ zu der Hautpartie und/oder der Probe auf das Testelement übertragen wird. In anderen Worten kann beispielsweise Blut zunächst in und/oder auf einer Hautpartie, beispielsweise eines Fingers, gesammelt werden, um anschließend beispielsweise direkt über ein Testfeld von der Hautoberfläche aufgenommen zu werden. Der Mechanismus kann beispielsweise analog zu dem in EP 1 992 283 A1 oder in EP 1 881 322 A1 beschriebenen Mechanismus ausgestaltet sein.

Ausgehend von einem derartigen Grundsystem, welches auf alle Aspekte der nachfolgend beschriebenen Erfindung anwendbar ist, beruht die Erfindung auf Untersuchungen zum Trocknungsverhalten von Blutproben. Diese Experimente wurden teilweise an offenen Proben durchgeführt, teilweise jedoch auch an Kapillaren, beispielsweise geöffneten Kapillaren in Nadeln. Diese Untersuchungen kommen im Wesentlichen zu dem Ergebnis, dass sich, wie oben beschrieben, theoretische oder semi-empirische Modelle, welche auf Untersuchungen an freien oder aufgesetzten Tropfen von Testflüssigkeiten wie beispielsweise Wasser basieren, nicht ohne Weiteres auf Systeme zum Nachweis von Analyten übertragen lassen, wie sie in der Praxis eingesetzt werden. Dementsprechend werden drei Konzepte vorgeschlagen, welche auch in Kombination einsetzbar sind und welche zur Vermeidung der oben dargestellten Problematik der Messungenauigkeit aufgrund von Verdunstungseffekten in Analysegeräten beziehungsweise den Systemen der oben beschriebenen Art eingesetzt werden können. Die Konzepte basieren auf demselben Grundgedanken, dass aufgrund der sehr vielfältigen Umgebungsbedingungen wie Druck, Feuchtigkeit, Temperatur, Konvektion oder ähnlichen Einflüssen im Bereich der Probe in herkömmlichen Systemen Korrekturen der Messergebnisse aufgrund der genannten analytischen oder semi-empirischen Modelle, beispielsweise im Rahmen von konstanten Korrekturfaktoren oder Korrekturfunktionen, nicht anwendbar sind, solange nicht zusätzliche Maßnahmen ergriffen werden.

Ein erstes Konzept der vorliegenden Erfindung besteht darin, den oben beschriebenen Transfer der Probe der Körperflüssigkeit auf das Testelement zeitlich zu begrenzen. So wurde herausgefunden, dass bei typischen und bevorzugten Probenvolumina im Bereich von unterhalb von 1 µl die Zeitdauer zwischen dem Generieren der Probe und dem Aufbringen auf das Testelement weniger als 1 s, vorzugsweise weniger als 800 ms, insbesondere weniger als 500 ms betragen sollte. Bei typischen Aufbauten und typischen Probenvolumina sowie typischen Testgeometrien führen derartige Transferzeiten von weniger als 1 s noch zu tolerablen Verfälschungen durch Verdunstungseffekte, beispielsweise Verfälschungen der Messergebnisse von weniger als 20 %, vorzugsweise von weniger als 5 %. Entsprechend kann das System gemäß einem ersten Aspekt der vorliegenden Erfindung derart eingerichtet sein, dass eine Zeitdauer zwischen dem Generieren der Probe und dem Aufbringen auf das Testelement weniger als 1 s, vorzugsweise weniger als 500 ms, beträgt. Besonders bevorzugt sind Transferzeiten von weniger als 200 ms oder sogar weniger als 100 ms. Unter der Transferzeit wird dabei hier und im Folgenden allgemein eine Zeitdauer zwischen einem ersten Kontakt eines den Probentransfer bewirkenden Elements mit einer Primärprobe (beispielsweise Körperflüssigkeit in und/oder auf einer Haut eines Probanden) bis hin zu einem ersten Kontakt der Probe mit dem mindestens einen Testelement, insbesondere der mindestens einen Testchemie, verstanden. Unter einer Primärprobe wird dabei die Probe in und/oder auf der Haut eines Probanden verstanden. Die Transferzeit kann auch in mehrere Zeitabschnitte unterteilt sein, beispielsweise eine Sammelzeit zum eigentlichen Aufnehmen der Probe durch das Transferelement (beispielsweise die Kapillare) und die Zeitdauer für den eigentlichen Transfer zum Testelement, welche auch als Transportzeit bezeichnet werden könnte. Die Sammelzeit und die Transportzeit können dabei auch überlappen, da beispielsweise ein Sammelvorgang während des eigentlichen Transfers nicht notwendigerweise abgeschlossen sein muss.

Diese Bedingung für die Transferzeit kann in dem System auf unterschiedliche Weisen gewährleistet sein, welche von der Art des Transfers abhängig ist. Beispielsweise kann eine der oben beschriebenen Transferarten in dem System gewährleistet sein. Beispielsweise kann eine Kapillare verwendet werden, insbesondere eine in eine Lanzette integrierte Kapillare. Die Kapillare kann geschlossen oder auch zumindest teilweise geöffnet, beispielsweise als zumindest teilweise offener Kanal mit grundsätzlich beliebigem Querschnitt, beispielsweise rechteckigem, rundem oder dreieckigem Querschnitt, ausgestaltet sein.

Insbesondere um in derartigen Systemen mit einer Kapillare, insbesondere einer geöffneten Kapillare, allgemein und ohne Beschränkung auf die weiteren oben beschriebenen Merkmale, vorzugsweise die oben beschriebenen Transferzeiten zu gewährleisten, ist es bevorzugt, wenn die Länge der Kapillare nicht mehr als 8 mm (Länge < 8 mm oder ≤ 8 mm), vorzugsweise nicht mehr als 6 mm (Länge < 6 mm oder ≤ 6 mm) und besonders bevorzugt nicht mehr als 4 mm (Länge < 4 mm oder ≤ 4 mm), beträgt. Beispielsweise können als Kapillaren Spalte mit einer Spaltbreite von 20 Mikrometern bis 500 Mikrometern, vorzugsweise zwischen 50 Mikrometern und 200 Mikrometern und besonders bevorzugt bei 100 Mikrometern verwendet werden. Die genannten bevorzugten Bedingungen gelten insbesondere für Systeme der oben beschriebenen Art, jedoch auch allgemein für andere Systeme zum Nachweis mindestens eines Analyten in einer Körperflüssigkeit, welche eingerichtet sind, um eine Probe der Körperflüssigkeit zu generieren und zumindest teilweise auf mindestens ein Testelement zu transferieren und bei denen für den Transfer der Probe mindestens eine Kapillare vorgesehen ist.

Es hat sich gezeigt, dass eine Füllgeschwindigkeit einer Kapillare von der Kapillarlänge und/oder Kapillargeometrie abhängig sein kann. Insbesondere kann die Füllgeschwindigkeit der Kapillare, also des für die Messung relevanten Kapillarabschnitts, exponentiell in Abhängigkeit der Kapillarlänge abnehmen. Um dennoch eine kurze Befüllzeit zu gewährleisten, weist eine besonders bevorzugte Kapillargeometrie ein Verhältnis der zu befüllenden Kapillarlänge zum Kapillardurchmesser auf, welches kleiner ist als 100, vorzugsweise kleiner ist als 30, insbesondere kleiner ist als 20 und besonders bevorzugt 15 oder weniger. Alternativ zum Kapillardurchmesser können auch andere, die Breite des Kapillarquerschnitts charakterisierende Dimensionen verwendet werden, beispielsweise bei einer geöffneten, insbesondere halb offenen Kapillare anstelle des Durchmesser auch die Länge der Bodenfläche, zuzüglich dem Doppelten der Höhe der Kapillarwände.

Zur Beschleunigung des Transfers, das heißt zur Verkürzung der Transferzeit, kann die mindestens eine Kapillare, sei sie nun als geschlossene Kapillare oder als zumindest teilweise geöffnete Kapillare ausgestaltet, weiterhin mindestens eine Hydrophilisierung umfassen. Beispielsweise kann es sich dabei um eine oder mehrere hydrophile Beschichtungen handeln. Beispielsweise können Beschichtungen mit Detergenzien eingesetzt werden. Insbesondere können eine oder mehrere der folgenden Materialien für die Hydrophilisierung eingesetzt werden: Heparin; Polyacrylsäure oder Polyacrylsäurederivate; Chondroitinsulfat; Dioctylnatriumsulphosuccinat (DONS); Polysorbat; nicht-ionische Tenside. Diesbezüglich kann beispielsweise auf die europäische Patentanmeldung mit der Anmeldenummer EP 07 114 414.1 verwiesen werden oder alternativ auf EP 1 887 355 A1. Alternativ oder zusätzlich können jedoch auch hydrophilisierende Oberflächenbehandlungen durchgeführt werden, wie beispielsweise hydrophilisierende Plasmabehandlungen, beispielsweise Sauerstoffplasma-Behandlungen oder Ähnliches. Auf diese Weise kann der Transfer der Probe zusätzlich beschleunigt werden, indem beispielsweise die Sammelzeit verkürzt wird. Die Transferzeit, welche zwischen dem Generieren der Probe auf einer Hautpartie und der Übertragung auf das Testelement beziehungsweise ein Testfeld des Testelements besteht, kann sich beispielsweise aus mehreren Bestandteilen zusammensetzen. So kann beispielsweise ein Teil dieser Transferzeit aus einer Sammelzeit und/oder Füllzeit der Kapillare bestehen, sowie anschließend beispielsweise einer Transportzeit zum Testelement, bis dieses mit dem Blut in Kontakt gebracht wird. Beispielsweise lassen sich auf diese Weise die oben beschriebenen Transferzeiten realisieren.

Auch bei Systemen, welche, alternativ oder zusätzlich zur Verwendung einer Kapillare, andere Transferkonzepte verwenden, sind die genannten Transferzeiten realisierbar. So kann beispielsweise das System, wie oben beschrieben, eingerichtet sein, um die Probe auf einer Hautoberfläche zu generieren, wobei das System weiterhin eingerichtet ist, um anschließend das Testelement relativ zur Hautoberfläche derart zu bewegen, dass das Testelement die Probe ganz oder teilweise aufnimmt. Dies kann beispielsweise mittels des oben beschriebenen Mechanismus erfolgen. Dabei kann die Probenaufnahme, beispielsweise durch entsprechende Ausgestaltung des Mechanismus, derart ausgestaltet sein, dass diese Probenaufnahme innerhalb der Zeitdauer von weniger als 1 s, insbesondere von weniger als 500 ms, besonders bevorzugt von weniger als 200 ms oder sogar 100 ms, erfolgt. Durch die Verwendung der genannten Transferzeiten, welche in den genannten bevorzugten Zeitfenstern liegen, lassen sich Verdunstungseffekte und deren Einfluss auf die Messgenauigkeit derart minimieren, dass die Messgenauigkeit innerhalb von üblicherweise bei beispielsweise Blutzuckermessungen vorgegebenen Toleranzen liegt, beispielsweise im Rahmen einer Toleranz von 20%. Bei Blutzuckermessungen werden beispielsweise typischerweise Toleranzen von 20% bei Konzentrationen von über 100 mg/dl vorgegeben, wohingegen unterhalb von 100 mg/dl Toleranzen von 20 mg/dl vorgegeben werden. Die Angaben sind jeweils darauf bezogen, dass jeweils 95% der Werte innerhalb des Toleranzintervalls liegen.

Alternativ oder zusätzlich zum Konzept der Beschleunigung der Transferzeit wird weiterhin ein Konzept vorgeschlagen, bei welchem gezielt Einfluss auf das Probenvolumen genommen wird. Wie oben dargestellt, werden bei modernen Blutglucose-Messgeräten Probenvolumina allgemein von unterhalb von 1 µl angestrebt. Überraschenderweise wurde im Rahmen der unten näher beschriebenen Untersuchungen, bei welchen festgestellt wurde, dass die Verdunstung deutlich größer ist als aus der Literatur anzunehmen wäre, jedoch herausgefunden, dass diese pauschale Minimierung der Probenvolumina ohne Untergrenze der Probenvolumina zu starken Problemen führen kann. Insbesondere wurde herausgefunden, dass Probenvolumina von 10 nl oder weniger derart starke Verdunstungseffekte aufweisen, dass in den meisten Fällen die durch die Verdunstung bedingte Messungenauigkeit den zumindest für Blutglucosemessgeräte tolerierbaren Rahmen überschreitet.

Erfindungsgemäß wird daher in einem weiteren Aspekt der vorliegenden Erfindung ein System gemäß der oben beschriebenen Art vorgeschlagen, bei welchem das Probenvolumen der Probe zwar kleiner ist als 500 nl, insbesondere kleiner als 400 nl, kleiner als 300 nl, kleiner ist als 200 nl oder sogar kleiner ist als 100 nl, jedoch größer ist als 10 nl. Vorzugsweise beträgt das Probenvolumen mindestens 12 nl.

Dabei wird als das Probenvolumen im Rahmen der vorliegenden Erfindung allgemein das Volumen der Probe bezeichnet, welches von dem System ursprünglich aufgenommen wird, also bevor Verdunstungseffekte eingetreten sind. Vorzugsweise wird dieses Probenvolumen vollständig auf das Testelement übertragen, wobei jedoch auch Anteile in übrigen Teilen des Systems verbleiben können, beispielsweise in einer Kapillare. Damit ist der Begriff des Probenvolumens zu unterscheiden vom Gesamtvolumen der Probe, welches generiert wird, beispielsweise Blut auf und/oder in einer Fingerkuppe, einem Ohrläppchen oder einer Hautpartie im Armbereich. Von diesem Gesamtvolumen der Probe wird lediglich das Probenvolumen vom System aufgenommen. Das Probenvolumen kann vorzugsweise, wie unten näher ausgeführt wird, vom System erfasst werden. Das so erfasste Probenvolumen wird im Rahmen der vorliegenden Erfindung auch als tatsächliches Probenvolumen bezeichnet.

Das Probenvolumen kann also insbesondere in einem Intervall zwischen 10 nl und 500 nl (also 10 nl ≤ Probenvolumen < 500 nl) liegen, bevorzugt in den Intervallen 10 nl < Probenvolumen ≤ 400 nl, 10 nl < Probenvolumen < 300 nl, 10 nl < Probenvolumen < 200 nl und besonders bevorzugt im Intervall 10 nl < Probenvolumen < 100 nl oder sogar im Intervall 10 nl < Probenvolumen < 50 nl. Die untere Intervallgrenze beträgt dabei vorzugsweise geringfügig mehr als 10 nl, beispielsweise mindestens 12 nl.

Dabei kann, wie oben dargestellt, der Transfer der Probe zu dem Testelement wiederum beispielsweise durch eine oder mehrere der genannten Arten erfolgen. Insbesondere ist wiederum der Transfer mittels mindestens einer Kapillare, insbesondere mittels mindestens einer zumindest teilweise geöffneten Kapillare, zu nennen. Insbesondere kann wiederum eine in eine Lanzette integrierte Kapillare, insbesondere eine zumindest teilweise geöffnete Kapillare, genannt werden. Wiederum kann die Lanzette insbesondere als Lanzette mit integriertem Testelement ausgestaltet sein, also als "Get and Measure"-Lanzette oder, was im Folgenden begrifflich gleich verwendet wird, als Microsampler. Alternativ oder zusätzlich kann das System jedoch wiederum auch mit einem Mechanismus ausgestaltet sein, bei welchem zunächst die Probe auf einer Hautpartie generiert wird und anschließend durch eine relative Bewegung des Testelements relativ zu der Hautpartie und/oder der Probe auf das Testelement übertragen wird. Bezüglich der möglichen Ausgestaltungen kann auf die obige Beschreibung verwiesen werden, welche, wie aufgeführt, für sämtliche genannte erfindungsgemäße Konzepte einsetzbar sind.

Da das Probenvolumen, wie oben dargestellt, eine erhebliche Rolle bei den beschriebenen Verdunstungseffekten spielt, wird erfindungsgemäß eine Kontrolle desselben vorgeschlagen. Dies kann beispielsweise dadurch gewährleistet sein, dass das System eingerichtet ist, um ein tatsächliches Probenvolumen der von dem System aufgenommenen Probe und/oder der auf das Testelement transferierten Probe zu erfassen. Wie oben dargestellt, ist das Probenvolumen zu unterscheiden von dem generierten Probenvolumen, beispielsweise dem Volumen eines Blutstropfens auf einer Hautoberfläche. Das tatsächliche Probenvolumen stellt somit einen aktuellen Messwert der von dem System aufgenommenen Probe und/oder der auf das Testelement transferierten Probe dar.

Der Nachweis des Analyten kann dann beispielsweise unter Berücksichtigung des tatsächlichen Probenvolumens durchgeführt werden. Beispielsweise können eine oder mehrere Korrekturfaktoren und/oder sonstige Korrekturen, wie beispielsweise Korrekturfunktionen, verwendet werden, um Messwerte, die als Ergebnis des Nachweises generiert werden, entsprechend auf das tatsächliche Probenvolumen zu korrigieren. Auf diese Weise lassen sich Probenvolumen-abhängige Verdunstungseffekte und dadurch bedingte Konzentrationsänderungen des mindestens einen Analyten zumindest teilweise ausgleichen. Hierdurch lassen sich beispielsweise Aufkonzentrationen der Probe durch Verdunstungseffekte zumindest teilweise ausgleichen und/oder korrigieren.

Die Erfassung des tatsächlichen Probenvolumens kann auf unterschiedliche Weisen erfolgen, wobei grundsätzlich beispielsweise beliebige physikalische und/oder chemische Messmethoden zum Einsatz kommen können. Beispielsweise kann diese Erfassung eine optische Erfassung beinhalten. So kann beispielsweise eine räumliche Ausdehnung der Probe auf optischem Wege erfasst werden, insbesondere eine räumliche Ausdehnung auf dem Testelement und/oder in einer Kapillare. Dies kann beispielsweise dadurch geschehen, dass Kontrastunterschiede zwischen der Probe und den umgebenden Materialien erfasst werden, welche auch durch entsprechende Einfärbung des Systems und/oder der mit der Probe in Kontakt kommenden Systemkomponenten gezielt verbessert werden können. Zur optischen Erfassung kann beispielsweise mindestens ein optischer Sensor vorgesehen sein, beispielsweise ein bildgebender Sensor, beispielsweise ein Halbleitersensor, sowie gegebenenfalls eine entsprechende Bildverarbeitung. Beispielsweise kann auf diese Weise die Größe eines Probenflecks auf einem Testfeld erfasst werden, wodurch wiederum beispielsweise auf das tatsächliche Probenvolumen, welches auf das Testelement transferiert wurde, geschlossen werden kann. Ähnliche Messprinzipien sind beispielsweise aus US 6,847,451 B2 bekannt, in welcher bei Verwendung eines Detektor-Arrays nur diejenigen Felder des Arrays verwendet werden, welche ausreichend mit Probe bedeckte Bereiche eines Testfeldes zeigen. Im Unterschied dazu kann beispielsweise im Rahmen der vorliegenden Erfindung unter Verwendung ähnlicher Techniken quantitativ auf das tatsächliche Probenvolumen geschlossen werden. Alternativ oder zusätzlich können auch andere optische Messprinzipien eingesetzt werden, beispielsweise Beugungsmessungen, Transmissionsmessungen, Absorptionsmessungen, Reflexionsmessungen, Fluoreszenzlichtmessungen oder Kombinationen der genannten und/oder anderer Arten optischer Messungen, aus denen sich auf das tatsächliche Probenvolumen schließen lässt. Beispielsweise lassen sich in einer Kapillare und/oder an einer sonstigen repräsentativen Stelle des Systems Absorptionsmessungen und/oder Transmissionsmessungen und/oder Reflektionsmessungen durchführen, aus denen sich das Produkt aus der Konzentration einer probenspezifischen Substanz, beispielsweise Hämoglobin, und einer Füllstandsvariablen, beispielsweise einer Füllhöhe einer Kapillaren, ermitteln lässt. Hieraus wiederum lässt sich das tatsächliche Probenvolumen absolut und/oder relativ bestimmen. Beispielsweise kann die Kapillare, insbesondere eine Innenoberfläche der Kapillare, auch ganz oder teilweise mit einer Aufrauung versehen sein, beispielsweise durch einen Ätzprozess. Diese Aufrauung kann beispielsweise eine Reflektivität der Oberfläche erhöht werden. Auf diese Weise kann beispielsweise eine optische Kontrastverstärkung realisiert werden, insbesondere bei metallischen Kapillaren. Die Reflektivität kann durch Aufrauung gezielt beeinflusst werden, um beispielsweise durch Absorptionsmessungen und/oder Transmissionsmessungen und/oder Reflektionsmessungen eine Füllstandsmessung und/oder eine Volumenbefüllungsmessung in der Kapillare durchzuführen oder zu erleichtern. Alternativ oder zusätzlich zu einer optischen Erfassung können jedoch auch andere Arten von Erfassungen und/oder Sensoren eingesetzt werden, beispielsweise elektrische Sensoren und/oder kapazitive Sensoren. Das Konzept der Erfassung des tatsächlichen Probenvolumens ist auf sämtliche der oben beschriebenen Transferkonzepte und/oder andere Arten von Transferkonzepten übertragbar.

Das Probenvolumen, welches von dem System aufgenommen wird und/oder auf das Testelement transferiert wird, kann auf verschiedene Weisen eingestellt werden. Beispielsweise können hier die Geometrien einer Lanzette und/oder einer Kapillare und/oder des Testelements eine Rolle spielen. So kann beispielsweise durch Einstellen einer Kapillarengeometrie Einfluss auf das aufgenommene Probenvolumen genommen werden. Andererseits kann beispielsweise durch die Ausgestaltung einer Lanzettenspitze und/oder durch eine Einstichtiefe der Lanzette das aufgenommene Probenvolumen beeinflusst werden, da beispielsweise eine Generierung einer höheren Menge an Probe zu einer erhöhten Menge aufgenommener Probe führen kann.

Das System kann, insbesondere in Zusammenhang mit der Erfassung des tatsächlichen Probenvolumens, jedoch auch in anders ausgestalteten Systemen, insbesondere eingerichtet sein, um das Probenvolumen aktiv zu steuern und/oder zu regeln. Dies kann insbesondere durch Einstellen einer Einstichtiefe einer Lanzette erfolgen. Eine Regelung kann dabei beispielsweise in Zusammenhang mit der Erfassung des tatsächlichen Probenvolumens vorgesehen sein. So kann das System beispielsweise eingerichtet sein, um das tatsächlich aufgenommene Probenvolumen zu erfassen. Anschließend kann, beispielsweise iterativ und/oder kontinuierlich in einem Regelvorgang, das Probenvolumen geregelt werden, beispielsweise indem die Einstichtiefe einer Lanzette und/oder eine Dauer eines Einstichvorgangs beeinflusst werden. Dies kann im Rahmen eines einzelnen Einstichvorgangs erfolgen oder auch im Rahmen einer Mehrfacheinstechung. Auf diese Weise kann insbesondere das oben beschriebene bevorzugte Probenvolumen sichergestellt werden.

Ein drittes, wiederum auch in Kombination mit einem oder beiden der oben beschriebenen Konzepte anwendbares Konzept, welches ebenfalls auf der Erkenntnis von Verdunstungseffekten basiert, beruht auf einer gezielten Berücksichtigung und/oder Kontrolle der Umgebungsbedingungen. Diese Idee basiert auf dem Grundgedanken, dass in realen Systemen zum Nachweis von Analyten die Umgebungsbedingungen stark schwanken können. Insbesondere können sich hier, wie oben beschrieben, unterschiedliche Geometrien, Luftfeuchtigkeiten, Drücke, Temperaturen, Luftbewegungen (wie beispielsweise Konvektion) oder ähnliche Einflüsse bemerkbar machen.

Um den Einfluss dieser Parameter, welche insbesondere einen Einfluss auf die Verdunstung haben können, besser erfassen zu können, wird im Rahmen des dritten Konzeptes vorgeschlagen, bei der Generierung der Probe und/oder bei dem Transfer der Probe zu dem Testelement eine Feuchtigkeit, beispielsweise eine absolute und/oder eine relative Luftfeuchtigkeit, zu erfassen. Der Nachweis des Analyten kann dann unter Berücksichtigung dieser Feuchtigkeit vorgenommen werden. Beispielsweise kann die Feuchtigkeit an einer oder mehreren Stellen innerhalb und/oder außerhalb des Systems erfasst werden, beispielsweise unter Verwendung eines oder mehrerer entsprechender Feuchtesensoren. Beispielsweise kann die Feuchtigkeit am Ort der Generierung der Probe und/oder an einer oder mehreren Stellen im Bereich des Probentransfers und/oder am Ort des mindestens einen Testelements, insbesondere am Ort des mindestens einen Testfeldes, bestimmt werden.

Vorzugsweise kann das System derart eingerichtet werden, dass der Einfluss von Schwankungen von Umgebungsparametern, wie beispielsweise Luftfeuchtigkeit, Drücken, Temperaturen, Luftbewegungen (wie beispielsweise Konvektion) oder ähnlichen Parametern zumindest weitestgehend ausgeschaltet wird, so dass Schwankungen verringert werden können, was wiederum die Berücksichtigung eines Einflusses dieser Parameter, insbesondere der Feuchtigkeit, beim Nachweis des mindestens einen Analyten erleichtert. Dementsprechend kann das System weiterhin derart eingerichtet sein, dass die Generierung der Probe und der Transfer zu dem Testelement innerhalb eines im Wesentlichen geschlossenen Gehäuses durchgeführt werden. Unter einem im Wesentlichen geschlossenen Gehäuse ist dabei ein Gehäuse zu verstehen, welches insoweit luftdicht und/oder feuchtigkeitsdicht ist, um einen Innenraum des Gehäuses gegenüber einer Umgebung des Systems abzuschließen. Diesbezüglich kann beispielsweise auf die EP 1 881 322 A1 und die in dieser Schrift dargestellten Möglichkeiten des Abschlusses eines Gehäuses verwiesen werden. Der Abschluss des Gehäuses soll zumindest derart erfolgen, dass zumindest während typischen Messdauern von beispielsweise nicht mehr als 5 bis 10 s, sich die Umgebungsbedingungen, beispielsweise hinsichtlich der oben genannten Parameter, im Innenraum des Gehäuses praktisch nicht ändern, so dass Änderungen dieser Parameter einen lediglich zu vernachlässigenden Einfluss auf die Verdunstungsrate beziehungsweise die Änderung der Verdunstungsrate aufweisen. Beispielsweise können Schwankungen in der Verdunstungsrate von nicht mehr als 5 % toleriert werden.

Das Gehäuse kann beispielsweise einteilig oder mehrteilig ausgestaltet sein und kann beispielsweise ein metallisches Gehäuse und/oder ein Kunststoffgehäuse umfassen. Das Gehäuse kann insbesondere eine oder mehrere Öffnungen umfassen, vorzugsweise mindestens eine verschließbare Öffnung. Diese Öffnung soll derart ausgestaltet sein, dass eine Hautpartie, insbesondere eine Hautpartie eines Fingers, ganz oder teilweise in die Öffnung einbringbar ist, wobei die Hautpartie dann die Öffnung zumindest teilweise, vorzugsweise vollständig, verschließt. Dieses Verschließen der Öffnung durch die Hautpartie kann während des gesamten Messvorgangs aufrechterhalten werden, so dass die oben beschriebene Abschottung des Innenraums des Gehäuses gegenüber der Umgebung erfolgt. Der Innenraum des Gehäuses kann, um die Bedingungen in diesem möglichst konstant zu halten, vorzugsweise möglichst klein gehalten werden, beispielsweise kleiner als 100 ml, insbesondere kleiner als 50 ml und besonders bevorzugt kleiner als 10 ml. Weiterhin kann, alsternativ oder zusätzlich, auch die Öffnung sehr klein gehalten werden, beispielsweise kleiner als 100 mm², insbesondere kleiner als 50 mm², vorzugsweise kleiner als 20 mm², 10 mm² oder kleiner. Die Generierung der Probe kann dann in der in die Öffnung eingebrachten Hautpartie erfolgen. Weiterhin erfolgt auch, wie oben beschrieben, der Transfer der Probe zu dem Testelement innerhalb des Innenraums des Gehäuses. Dieser Transfer kann wiederum beispielsweise mittels der oben beschriebenen Konzepte erfolgen. So kann beispielsweise wiederum mindestens eine Kapillare verwendet werden, insbesondere eine zumindest teilweise in eine Lanzette integrierte Kapillare. Insbesondere kann wiederum eine Lanzette mit integriertem Testelement verwendet werden. Alternativ oder zusätzlich kann jedoch wiederum auch ein Verfahren verwendet werden, bei welchem zunächst die Probe auf der Hautpartie generiert wird und anschließend zumindest teilweise durch eine relative Bewegung des Testelements relativ zu der Hautpartie und/oder der Probe auf das Testelement übertragen wird. Zu diesem Zweck kann wiederum ein Mechanismus vorgesehen sein. Auch Kombinationen der genannten Transferkonzepte und/oder anderer Transferkonzepte sind möglich.

Ist mindestens eine Öffnung vorgesehen, so kann diese beispielsweise mittels eines Schließmechanismus verschließbar sein, während das System nicht zur Messung verwendet wird. Beispielsweise kann die Öffnung mittels mindestens eines Schiebers, einer Klappe, einer flexiblen Dichtlippe oder ähnlicher Verfahren verschlossen sein, so dass die Öffnung zum Durchführen einer Messung geöffnet werden kann. Alternativ lassen sich jedoch auch Öffnungen einsetzen, welche während einer Ruhephase, in welcher keine Messung durchgeführt wird, geöffnet verbleiben. Erst während der Messung, wenn die Öffnung durch die Hautpartie verschlossen wird, werden dann durch das Gehäuse vorzugsweise im Wesentlichen konstante Umgebungsbedingungen innerhalb des Innenraums des Gehäuses gewährleistet.

Wie oben dargestellt, wird vorgeschlagen, bei der Generierung der Probe und/oder dem Transfer der Probe zu dem Testelement eine Feuchtigkeit zu erfassen, insbesondere innerhalb des Gehäuses. Zu diesem Zweck können ein oder mehrere Feuchtigkeitssensoren vorgesehen sein, welche eine absolute und/oder relative Luftfeuchtigkeit der Atmosphäre an einer oder mehreren der oben genannten Stellen, beispielsweise im Inneren des Gehäuses, erfassen können. Das System kann dann insbesondere eingerichtet sein, um den Nachweis des Analyten unter Berücksichtigung der mindestens einen Feuchtigkeit durchzuführen. Dabei können beispielsweise, wenn mehrere Feuchtigkeit gemessen werden, diese einzeln oder auch in Kombination, beispielsweise in Form von Mittelwerten, berücksichtigt werden. Die Berücksichtigung der tatsächlich vorhandenen Feuchtigkeit, insbesondere der Luftfeuchtigkeit, welche aufgrund der beschriebenen bevorzugten Kapselung durch das Gehäuse vergleichsweise geringen und vernachlässigbaren Schwankungen unterworfen ist, kann beispielsweise unter Verwendung eines bekannten Einflusses der Luftfeuchtigkeit auf eine Verdunstungsrate erfolgen. So können beispielsweise Korrekturfaktoren und/oder Korrekturfunktionen und/oder andere Arten von Korrekturen verwendet werden, bei welchen, beispielsweise unter Berücksichtigung der tatsächlich im System auftretenden Geometrien, Verdunstungseffekte bei der aktuellen Luftfeuchtigkeit und die damit beschriebenen Aufkonzentrierungen der Probe korrigiert werden. Die Korrekturen können beispielsweise auf analytischen, semi-empirischen oder empirischen Erkenntnissen über die Verdunstung beruhen.

Im Gegensatz zu bekannten Systemen und/oder theoretischen Ansätzen ist eine Korrektur in dem vorgeschlagenen System gemäß dem dritten Konzept der Erfindung auf einfache Weise realisierbar. Dies ist dadurch bedingt, dass die unbekannten Einflüsse, welche in herkömmlichen Systemen die Korrektur verhindern oder zumindest erschweren, im vorgeschlagenen System zumindest teilweise bekannt sind und vorzugsweise auch weitgehend ausgeschaltet werden. Durch die Kapselung durch das optionale Gehäuse wird beispielsweise eine Konvektion und/oder eine Änderung der Konvektionsbedingungen während der Messung weitgehend verhindert. Auch Schwankungen der Luftfeuchtigkeit können ausgeschlossen werden.

Weiterhin kann das System auch eingerichtet sein, um den Nachweis des Analyten bei Unterschreiten einer vorgegebenen Mindestfeuchtigkeit zumindest vorübergehend zu unterbrechen. So kann beispielsweise ein Abbruch der Messung erfolgen und/oder eine Warnung generiert werden. Beispielsweise können eine oder mehrere Feuchtigkeitsschwellen vorgegeben sein, welche mit dem aktuellen Messwert der Luftfeuchtigkeit verglichen werden. Beispielsweise kann auf diese Weise festgestellt werden, dass eine Verdunstung aufgrund einer zu geringen Luftfeuchtigkeit im aktuellen Fall zu groß wäre, so dass die damit verbundene Beeinflussung des Nachweises des Analyten einen Toleranzbereich überschreiten würde. In diesem Fall kann beispielsweise eine Warnung an einen Benutzer ausgegeben werden, die Messung zu einem späteren Zeitpunkt und/oder unter anderen Umgebungsbedingungen zu wiederholen. Alternativ oder zusätzlich kann auch ein Benutzer beispielsweise aufgefordert werden, durch eine Öffnung in das Innere des Gehäuses zu blasen bzw. zu hauchen, um durch die Atemluft eine Feuchtigkeit im Inneren des Gehäuses und/oder an anderen Stellen gezielt zu erhöhen.

Das System kann auch weiterhin eingerichtet sein, um mindestens einen weiteren Parameter bei der Generierung der Probe und/oder dem Transfer der Probe zu dem Testelement, insbesondere innerhalb des Gehäuses, zu erfassen. Insbesondere kann es sich dabei um einen Parameter handeln, welcher Einfluss auf die Verdunstung beziehungsweise die Verdunstungsrate der Probe und/oder von Bestandteilen der Probe hat. Beispielsweise können ein Druck und/oder eine Temperatur erfasst werden, beispielsweise ein Druck im Inneren des Gehäuses und/oder eine Temperatur eines Testelements und/oder der Lanzette und/oder eine Lufttemperatur, insbesondere innerhalb des Gehäuses. Dieser mindestens eine weitere Parameter kann ebenfalls vorzugsweise beim Nachweis des Analyten in der Probe berücksichtigt werden, beispielsweise durch entsprechende Korrekturen, analog zur oben beschriebenen Korrektur unter Kenntnis der Luftfeuchtigkeit.

Das vorgeschlagene System, welches beispielsweise ganz oder teilweise als tragbares Messgerät ausgestaltet sein kann und/oder als stationäres Messgerät, weist gegenüber bekannten Systemen eine Vielzahl von Vorteilen auf. So können durch die oben beschriebenen Konzepte Einflüsse der Verdunstung entweder gezielt beeinflusst werden (beispielsweise durch Erfassung und/oder Steuerung des Probenvolumens) und/oder zumindest so weit kontrolliert werden, dass Schwankungen dieser Einflüsse durch Änderung der Umgebungsbedingungen und/oder der Probennahmebedingungen zumindest weitgehend eliminiert werden können. Beispielsweise kann durch das oben beschriebene Erfassen des tatsächlichen Volumens der Probe eine Verdunstungsrate angenommen werden, beispielsweise unter gleichzeitiger Berücksichtigung einer gemessenen Feuchtigkeit im Inneren eines Gehäuses. Auf diese Weise kann gezielt beispielsweise eine Korrektur der Messung vorgenommen werden, welche beispielsweise den Betrag der verdunsteten Probe berücksichtigt. Eine Realisierung einer derartigen Korrektur ist insbesondere dann technisch auf einfache Weise zu realisieren, wenn, wie oben beschrieben, der vollständige Vorgang im Inneren eines Gehäuses stattfindet, welches eine Abschirmung der beschriebenen Art bereitstellt. Beispielsweise können ein Stechvorgang sowie ein Bluttransfer auf das Testelement vollständig innerhalb des Systems, also im Gehäuseinneren erfolgen, so dass von einer konstanten Luftfeuchtigkeit während des Messablaufs ausgegangen werden kann.

### Kurzbeschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigt:
- Figur 1: ein schematisches Ausführungsbeispiel eines erfindungsgemäßen Systems;
- Figur 2: ein aus der Literatur bekannter Zusammenhang zwischen einer Verdunstungsrate und einer Öffnungsfläche einer Flüssigkeit;
- Figur 3: eine Messung einer relativen Abweichung einer gemessenen Glucosekonzentration vom Mittelwert als Funktion der Dauer des Zeitintervalls zwischen Probennahme und Probenauftrag;
- Figur 4: typische Füllzeiten unterschiedlich beschichteter Kapillaren;
- Figur 5: extrapolierte Daten einer Verdunstungszeit als Funktion der relativen Luftfeuchte in Prozent; und
- Figuren 6A und 6B: Oberflächenaufnahmen an metallischen Kapillaren ohne Aufrauung und mit Aufrauung.

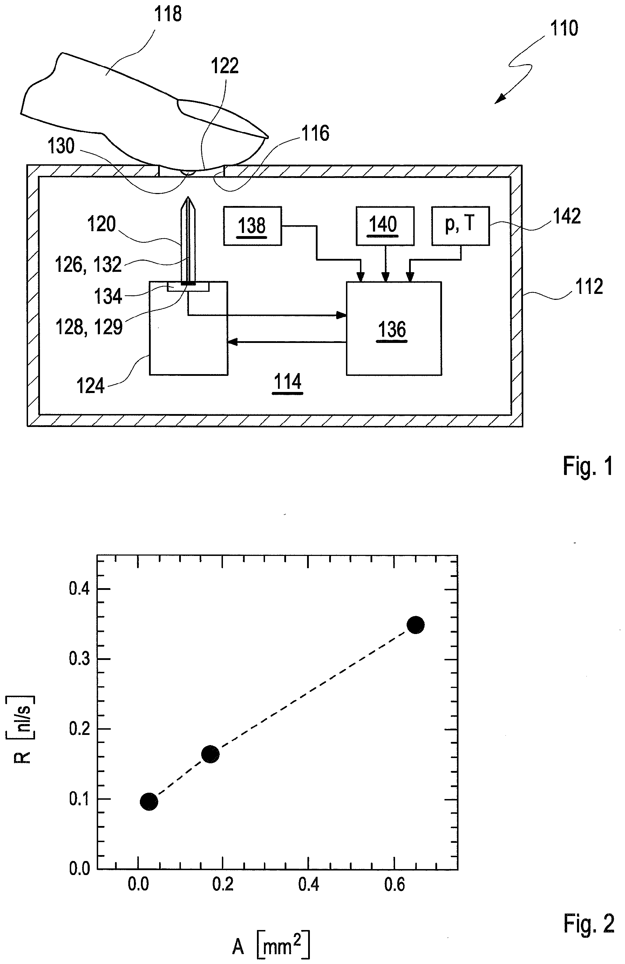

In Figur 1 ist ein stark schematisiertes Ausführungsbeispiel eines erfindungsgemäßen Systems 110 zum Nachweis mindestens eines Analyten in einer Körperflüssigkeit dargestellt. Das System 110 umfasst in dem dargestellten Ausführungsbeispiel ein im Wesentlichen feuchtigkeitsdichtes Gehäuse 112, welches im Wesentlichen geschlossen ausgestaltet ist. Das Gehäuse 112 umfasst einen Innenraum 114, welcher im dargestellten Ausführungsbeispiel durch eine Öffnung 116 in dem Gehäuse 112 für eine Messung vorübergehend zugänglich ist. Die Öffnung 116 kann beispielsweise mittels eines in der Figur 1 nicht dargestellten Schiebers verschließbar ausgestaltet sein, wobei der Schieber beispielsweise mit einem Finger 118 eines Patienten oder sonstigen Benutzers für eine Messung geöffnet werden kann.

Im Innenraum 114 ist eine Lanzette 120 angeordnet, welche eingerichtet ist, um bei auf die Öffnung 116 aufgelegtem Finger 118, wobei der Finger 118 die Öffnung 116 vollständig oder zumindest teilweise verschließt, eine Hautpartie 122 im Bereich einer Kuppe des Fingers 118 zu perforieren. Die Lanzette 120 kann beispielsweise durch einen Aktor 124 zu einer Stechbewegung angeregt werden.

Weiterhin umfasst das System 110 im dargestellten Ausführungsbeispiel eine Transfervorrichtung 126 sowie mindestens ein Testelement 128 zum Nachweis des Analyten in einer durch die Lanzette 120 auf der Hautpartie 122 generierten Probe 130, im vorliegenden Fall eines Tropfens von Blut oder interstitieller Flüssigkeit. Dabei ist im dargestellten Ausführungsbeispiel die Transfervorrichtung 126 als Kapillare 132 ausgestaltet, welche beispielsweise als Spalt in der Lanzette 120 geformt sein kann. Mittels dieser Kapillare 132 wird die Probe 130 vollständig oder teilweise zu dem Testelement 128 transferiert, welches in diesem Fall beispielsweise einstückig mit der Lanzette 120 ausgebildet sein kann. Die Lanzette 120 kann also beispielsweise als so genannter Microsampler oder "Get and Measure"-Lanzette ausgestaltet sein.

Das Testelement 128 kann beispielsweise ein Testfeld 129 umfassen, welches am Ende der Kapillare 132 angeordnet ist. Weiterhin kann eine Messvorrichtung 134 vorgesehen sein, welche, beispielsweise elektrisch und/oder optisch, mit dem Testelement 128 gekoppelt ist, um den mindestens einen Analyten in der Probe 130 nach Transfer auf das Testelement 128 nachzuweisen.

Es wird darauf hingewiesen, dass die in Figur 1 gezeigte Art des Transfers der Probe 130 auf das Testelement 128 beziehungsweise das Testfeld 129 lediglich eine von mehreren, auch in Kombination realisierbaren Transfermöglichkeiten darstellt. Beispielsweise kann, wie oben beschrieben und beispielsweise aus EP 1 992 283 A1 oder EP 1 881 322 A1 bekannt, auch zunächst mittels der Lanzette 120 eine Probe 130 auf der Hautpartie 122 generiert werden, wonach diese, beispielsweise durch eine entsprechende Bewegung des Testelements 128, von der Hautpartie 122 abgeholt wird und auf das Testelement 128 übertragen wird.

Weiterhin kann das System 110 eine Steuerung 136 umfassen. Diese Steuerung 136 kann beispielsweise mit der Messvorrichtung 134 ganz oder teilweise identisch sein, kann jedoch auch, wie in Figur 1 gezeigt, von dieser getrennt ausgebildet sein und mit dieser verbunden sein. Die Steuerung kann weiterhin mit dem Aktor 124 verbunden sein und diesen beispielsweise ansteuern. Die Steuerung 136 kann beispielsweise auch eine oder mehrere Datenverarbeitungsvorrichtungen umfassen, welche den gesamten Messablauf des Systems 110 steuern können und/oder eine Auswertung der Messung des mindestens einen Analyten vornehmen kann. Alternativ oder zusätzlich können auch andere elektronische Auswertevorrichtungen in der Steuerung 136 vorgesehen sein. Die Steuerung 136 kann beispielsweise auch mit einer oder mehreren Ein- und Ausgabemitteln versehen sein, beispielsweise Bedienelementen, Anzeigeelementen oder Ähnlichem, um eine Einflussnahme eines Benutzers auf die Systemsteuerung 110 und/oder eine Ausgabe von Informationen an den Benutzer zu ermöglichen. Für die Ausgestaltung derartiger Ein- und Ausgabemittel kann beispielsweise auf herkömmliche Blutglucose-Messgeräte verwiesen werden. Die Steuerung 136 kann auch beispielsweise einen oder mehrere Datenspeicher umfassen, beispielsweise flüchtige und/oder nicht-flüchtige Datenspeicher, welche gegebenenfalls auch mit einem Datenbanksystem zur Speicherung von Messwerten ausgestattet sein können. Die Steuerung 136 kann beispielsweise programmtechnisch eingerichtet sein, um die oben beschriebenen Verfahren in einer oder mehreren der beschriebenen Varianten durchzuführen, also beispielsweise eine Berücksichtigung von Verdunstungseffekten und/oder eine Korrektur derartiger Effekte bei der Auswertung des Nachweises des mindestens einen Analyten.

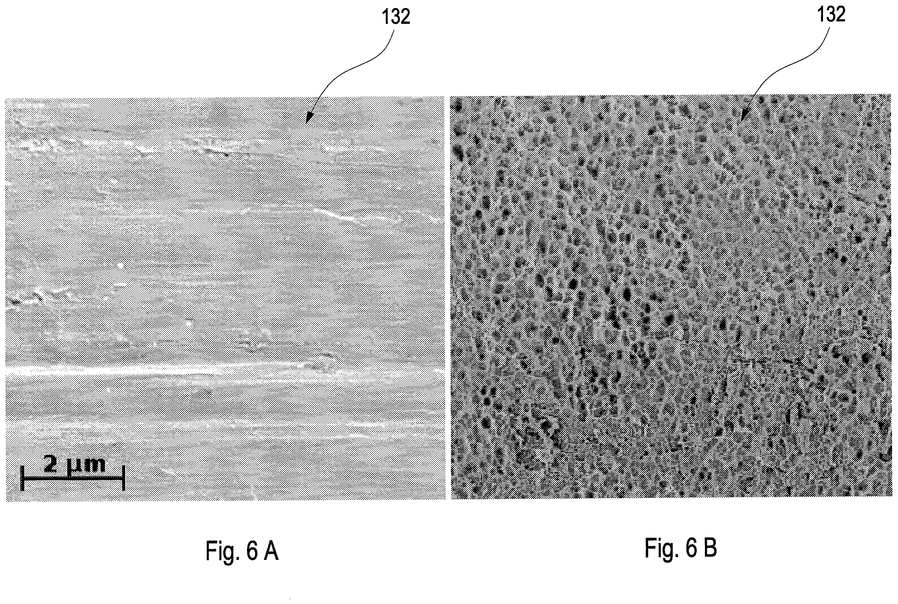

Weiterhin umfasst das System 110 gemäß Figur 1 eine Mehrzahl von Sensoren. So kann beispielsweise ein optischer Sensor 138 vorgesehen sein, welcher ein tatsächliches Probenvolumen einer aufgenommenen Probe 130 erfassen kann und welcher beispielsweise mit der Steuerung 136 verbunden sein kann, um eine Information über dieses tatsächliche Probenvolumen an die Steuerung 136 zu übermitteln. Der optische Sensor 138 kann beispielsweise mittels einer Reflexionsmessung und/oder anderen optischen Messverfahren einen Füllstand und/oder eine Volumenbefüllung der Kapillare 132 bestimmen. Zu diesem Zweck kann die Kapillare 132 beispielsweise auf besondere Weise ausgestaltet sein, insbesondere um eine Reflexionsmessung zu erleichtern. Die Kapillare 132 kann beispielsweise mit einer Aufrauung versehen sein, um eine Reflexionsmessung zu erleichtern. Dies ist exemplarisch in den Figuren 6A und 6B dargestellt, welche Oberflächenaufnahmen von metallischen Oberflächen zeigen. Beispielsweise kann die Kapillare 132 aus einem metallischen Material hergestellt sein, beispielsweise einem Metallblech, beispielsweise Stahl. Figur 6A zeigt eine Aufnahme einer unbehandelten metallischen Oberfläche, wohingegen Figur 6B eine Aufnahme einer mittels eines Ätzverfahrens aufgerauten metallischen Oberfläche zeigt. Beispielsweise kann ein Kapillarkanal der Kapillaren 132 auf diese Weise vollständig oder teilweise aufgeraut werden, insbesondere um gezielt eine des Kapillarkanals einstellen zu können. Wird eine Information über das tatsächliche Probenvolumen erhalten, beispielsweise mittels des Sensors 138, so kann die Steuerung 136 insbesondere eingerichtet sein, um die Auswertung der Messung des mindestens einen Analyten unter Berücksichtigung dieser Information vorzunehmen.

Weiterhin kann das System 110 mindestens einen Feuchtigkeitssensor 140 umfassen, welcher ebenfalls mit der Steuerung 136 verbunden sein kann und welcher eine Feuchtigkeit im Innenraum 114 messen kann. Die Steuerung 136 kann wiederum eingerichtet sein, um die Auswertung der Messung unter Berücksichtigung dieser Feuchtigkeitsinformation vorzunehmen. Weiterhin können im Innenraum 114 und/oder außerhalb des Innenraums 114, ein oder mehrere weitere Sensoren 142 für die Messung weiterer Parameter vorgesehen sein. Beispielsweise können, wie in Figur 1 angedeutet, ein oder mehrere Sensoren für einen Druck, eine Temperatur oder ähnliche Parameter vorgesehen sein. Auch diese Sensoren 142 können mit der Steuerung 136 verbunden sein, so dass die Auswertung der Messung unter Berücksichtigung der zusätzlichen Parameter erfolgen kann.

Zur Untersuchung der Verdunstungsproblematik, welche Einfluss auf den Nachweis des mindestens einen Analyten in der Probe 130 haben kann, wurden verschiedene aus der Literatur bekannte Studien ausgewertet. So ist während des Ansaugens von Blut, beispielsweise durch eine offene Mikrokapillare, mit einer teilweisen Verdunstung des Blutwassers zu rechnen, noch bevor dieses das Testelement 128, beispielsweise das Testfeld 129, erreicht. Es sei darauf hingewiesen, dass anstelle eines einzelnen Testfeldes 129 auch andere Arten von Testelementen 128 verwendet werden können, beispielsweise Teststreifen, Testbänder, Testscheiben oder ähnliche, beispielsweise aus dem Stand der Technik verwendete Testelemente. Die im Folgenden beschriebenen Untersuchungen beziehen sich jedoch im Wesentlichen auf Mikrokapillaren, sind jedoch ohne Weiteres auch auf andere Arten von Systemen übertragbar.

Durch die beschriebene Verdunstung kommt es in der Regel unweigerlich zu einer Erhöhung der Konzentration sämtlicher gelöster Analyten. Dies bewirkt in der Regel einen Messfehler, der bereits durch die Probenentnahme auf dem Transportweg verursacht wird. Durch Kenntnis der zu erwartenden Verdunstungsrate kann jedoch der zu erwartende Fehler zumindest abgeschätzt werden. Eine Problematik besteht jedoch darin, dass die Funktionsfähigkeit über einen möglichst weiten Temperatur- und Funktionsbereich des Systems 110 gewährleistet sein sollte. Daher ist eine pauschale Korrektur, beispielsweise um 5 %, nicht ausreichend, da die Verdunstungseffekte stark variieren können. Sofern der Ansaugvorgang der Probe 130 beziehungsweise der Transfervorgang immer schnell genug verläuft, zum Beispiel innerhalb von 1 s abgeschlossen ist, wäre zumindest der Zeitfaktor nicht auch noch als variabler Parameter zu berücksichtigen.

Die Verdunstung, das heißt der Übergang flüssiger Teilchen in die Dampfphase unterhalb des Siedepunkts, ist ein diffusionslimitierter Vorgang, welcher in der Literatur verschiedentlich beschrieben worden ist. Die treibende Kraft der Verdunstung ist der Konzentrationsgradient des Dampfdrucks, beispielsweise des Wasserdampfdrucks, zwischen der Oberfläche der Probe 130 und weit entfernter Umgebung. Der Gradient ist umso steiler, je trockener, also aufnahmefähiger, die Umgebungsluft ist. In ruhender Luft bildet sich dieser Gradient infolge der Verdunstung allmählich aus.

In bewegter Luft hingegen hat der Gradient keine Gelegenheit, sich geometrisch geformt auszubilden. Bei gegebenem Anstand wird somit mit Luftzug, also bei bewegter Luft, der Konzentrationsgradient über der Flüssigkeit an seinem Maximum aufrechterhalten, während er bei stehender Luft durch die zunehmende Erhöhung der Luftfeuchtigkeit über der Flüssigkeit abnimmt. Nach einer anderen Betrachtungsweise desselben Umstands wird die Diffusionsgrenzschicht zunehmend geringer und damit der Gradient steiler. Daher wird in dem erfindungsgemäßen System 110 gemäß Figur 1 jegliche Luftbewegung durch Vorsehen des abgeschlossenen Gehäuses 112 vermieden, so dass sowohl die Generierung der Probe 130 als auch deren Transfer und Messung durch das Testelement 128 im Innenraum 114 erfolgt. Auf diese Weise lässt sich der Einfluss von Schwankungen durch Bewegungen der Luft auf die Verdunstungsrate zumindest insoweit konstant halten, dass dieser, im Gegensatz zu herkömmlichen Systemen, ebenfalls konstant gehalten werden kann. Auf diese Weise lassen sich theoretische oder semiempirische Ansätze, um den Einfluss der Konvektion auf die Verdunstungsrate zu korrigieren, vermeiden.

Die Verdunstung wird, neben der störenden Luftunruhe beziehungsweise Konvektion, darüber hinaus durch eine Vielzahl von Parametern beeinflusst. Unter Parametern werden dabei im Rahmen der vorliegenden Erfindung sämtliche Einflussgrößen zusammengefasst, welche Einfluss auf die Verdunstung haben können. Dabei kann es sich um Umgebungsparameter handeln, wie beispielsweise den Luftdruck, die Lufttemperatur, die Luftfeuchtigkeit, die Temperatur von Teilen des Systems 110, eine Konzentration von Analyten in der Probe 130 (welche beispielsweise über eine Dampfdruckerhöhung Einfluss nehmen können) oder sonstige Parameter oder Kombinationen der genannten und/oder anderer Parameter handeln. Als System-inhärente Parameter kommen darüber hinaus insbesondere Oberflächenbeschaffenheiten einzelner Teile des Systems 110, beispielsweise der Kapillare 132, Geometrien einzelner Teile des Systems 110, beispielsweise wiederum der Kapillare 132, oder sonstiger Bestandteile in Betracht.

In Figur 2 ist ein aus der Literatur bekannter Zusammenhang zwischen einer Verdunstungsrate R, angegeben in nl/s, und einer Öffnungsfläche A, angegeben in mm², in einer pyramidenförmigen, geätzten Vertiefung in Silizium dargestellt. Die gezeigte Messung ist aus Mayer et al., 1997, Sens. Actuators A, 60, 202-207, entnommen. Die Messungen wurden hier mit einem Probenvolumen von ca. 8 nl durchgeführt. Die Situation dieser Messungen ist zumindest näherungsweise mit der Verdunstung aus halboffenen Kapillaren, wie beispielsweise der Kapillare 132, vergleichbar. Die Messungen in Figur 2 wurden dabei an Wasser durchgeführt, bei einer Temperatur von 22°C und einer relativen Luftfeuchtigkeit von ca. 50 %.

Die Messung in Figur 2 zeigt, dass die Verdunstungsrate zumindest näherungsweise proportional zur Oberfläche A ist. Der oberste Wert in Figur 2 entstammt Mikrobehältern mit einer Oberfläche von 0,64 mm², was der Oberfläche offener Kanäle in Flachlanzetten, welche ca. 1 mm² beträgt, am nächsten kommt. Aus der genannten Veröffentlichung von Mayer et al. lassen sich für derartige Flachlanzette mit einer Oberfläche von 1 mm² Verdunstungsraten von ca. 0,5 nl/s extrapolieren. 100 nl Wasser wären demnach in ca. 200 s verdunstet.

Bei dem in der Literaturstelle von Mayer et al. verwendeten Volumen von ca. 90 bis 140 nl entspräche dies einer anfänglichen Verdunstung und damit Konzentrationsänderung von ca. 0,2 bis 0,3 %/s, also einem Wert, der sehr klein ist gegenüber der etablierten Messgenauigkeit, beispielsweise von Blutglucosemessgeräten. Man sollte daher aus diesen Literaturstellen erwarten, dass das Problem der Verdunstung bei Mikrokapillaren keine Rolle spielt.

Um diese Vorhersagen der Literatur zu verifizieren, wurden eigene Experimente zur Verdunstung aus einer Kapillare 132 durchgeführt. Die Ergebnisse sind in Tabelle 1 aufgezeigt.

**Tabelle 1: Messergebnisse realer Verdunstungsraten**

| | Tropfen | | Kapillare | |
|---|---|---|---|---|
| Volumen | Wasser | Blut | Wasser | Blut |
| 150 nl | 0,6 µg/s | 0,5 µg/s | - | - |
| 50 nl | 0,5 µg/s | - | 1,0 µg/s | 1,0 µg/s |

Gezeigt sind dabei jeweils Verdunstungsraten in µg/s für Wasser und für Blut. Beim Tropfen betrug dabei das Auftragsvolumen bei Blut 500 nl, wohingegen bei der Kapillare das Auftragsvolumen bei 250 nl betrug. Die Raten wurden jedoch jeweils bei den in der ersten Spalte angegebenen Werten bestimmt. Dabei sind Messergebnisse mit einem anfänglichen Tropfenvolumen von 150 nl und 50 nl gezeigt, welche sowohl an Tropfen als auch innerhalb einer geöffneten Kapillare mit einer Oberfläche von ca. 1 mm² erzielt wurden.

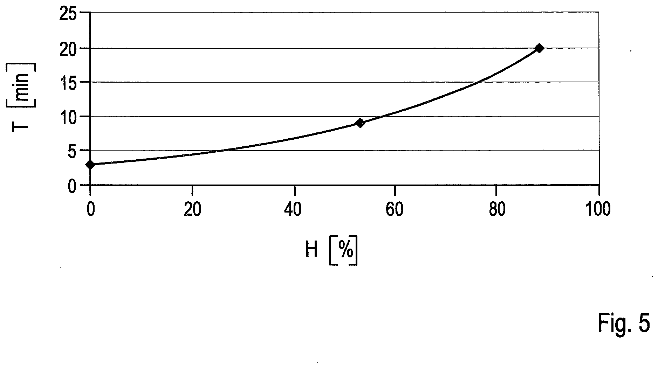

Überraschend zeigen diese Ergebnisse, dass die Verdunstung deutlich größer ist als aus der oben genannten Literatur anzunehmen wäre. 1 µg entspricht in etwa 1 nl Wasser. Die Messungen wurden bei 22°C und bei einer etwas geringeren Luftfeuchtigkeit als in der Literatur (45 %) aufgenommen. Die leicht verringerte Luftfeuchtigkeit kann jedoch nicht für den sehr großen Unterschied zu den Erwartungswerten verantwortlich gemacht werden, da die relative Luftfeuchtigkeit in diesem Bereich bei einer Änderung um ca. 5 % lediglich, wie Untersuchungen gezeigt haben, die Verdunstungsrate um maximal 20 bis 30 % beeinflussen können. Dies geht beispielsweise aus der Darstellung in Figur 5 hervor, in welcher die Verdunstungszeit T in Minuten für einen Wassertropfen mit 0,5 mm Durchmesser als Funktion der relativen Luftfeuchte H in % aufgetragen ist. Auch diese Darstellung ist aus Mayer et al., 1997, Sens. Actuators A, 60, 202-207, entnommen.

Bei den in Tabelle 1 gezeigten Messungen wurde die Konvektion durch Verwendung einer gekapselten Waage minimiert. Zwar wurde bei der in der Tabelle 1 gezeigten Messreihe untypischerweise eine Kapillare mit einer Länge von 8 mm verwendet, so dass sich die Oberfläche auf ca. 1 mm² und damit die Verdunstungsrate in Extrapolation der Figur 2 auf 0,6 nl/s vergrößert. Auch könnte argumentiert werden, dass ein aufgesetzter Tropfen gleichen Volumens (80 nl) eine Oberfläche von 0,7 mm² aufweist, da nur eine Hälfte des Tropfens mit der Umgebung in Kontakt kommt. Beide Betrachtungen können aber bei weitem nicht die Diskrepanz der literaturbasierten Erwartung (ca. 0,6 nl/s) zu den Messwerten in Tabelle 1 erklären, welche bei Verdunstungsraten von 1,0 nl/s liegen. Erfindungsgemäß wird daher vorgeschlagen, den in weiten Bereichen nur schwer vorhersehbaren und kontrollierbaren Einfluss der Verdunstung auf die Messergebnisse der Analytbestimmung durch verschiedene Maßnahmen zu vermindern und/oder konstant und damit korrigierbar zu halten und/oder diesen Einfluss durch entsprechende Regelungsmaßnahmen beziehungsweise Korrekturmaßnahmen gezielt zu eliminieren.

Eine Maßnahme besteht in der oben bereits beschriebenen Kapselung des Systems 110 durch das Gehäuse 112, vorzugsweise ein von der Lanzette 120 und/oder dem Aktor 124 unabhängig ausgebildetes Gehäuse 112, wodurch die Verdunstung durch Konvektion minimiert wird.

Eine andere Maßnahme besteht darin, den Zeitraum zwischen Probengenerierung und Probenauftrag auf das Testelement 128 sehr kurz zu halten, bevorzugt bei weniger als 1 s.

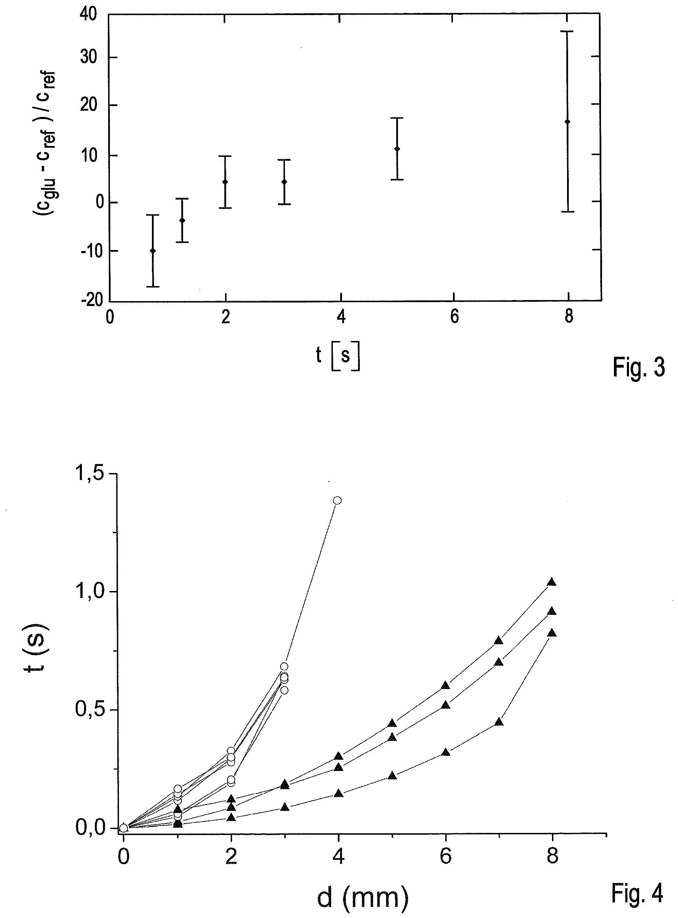

So sind in Figur 3 relative gemessene Abweichungen einer Glucosekonzentration von einem Mittelwert der Messreihe (c_{glu}-c_{ref})/c_{ref} als Funktion der Zeitdauer t in Sekunden des Zeitintervalls zwischen Probengenerierung und Probenauftrag auf das Testelement 128, also als Funktion der Transferzeit, dargestellt. Die Versuche wurden dahingehend vorgenommen, dass eine Kapillare 132 mit einer Probe 130 kontaktiert wurde und anschließend die gefüllte Kapillare händisch mit einem Testelement 128 in Form eines Teststreifens in Kontakt gebracht wurde. Der Versuchsaufbau war dabei nicht gekapselt. Dadurch wurde der Einfluss auf die gemessene Glucosekonzentration untersucht.

Die in Figur 3 dargestellte Messreihe zeigt deutlich eine systematische Veränderung des gemessenen Glucosewerts mit der Dauer des Zeitintervalls zwischen Probennahme und Probenauftrag auf den Teststreifen. Insbesondere ist ersichtlich, dass sich signifikante Abweichungen von einem anfänglichen Wert bereits innerhalb von 1 s ergeben. Dies zeigt, dass das genannte Zeitintervall zwischen Probengenerierung und Test durch das Testelement 128 vorzugsweise kürzer sein sollte als 1 s, wenn auf aufwendige Kapselungsmaßnahmen der Kapillare 132 verzichtet werden soll.

Verschiedene Möglichkeiten, um diese Transferzeit zwischen der Generierung der Probe 130 und der Kontaktierung des Testelements 128 zu beeinflussen, wurden oben bereits diskutiert. In Figur 4 ist exemplarisch eine Möglichkeit gezeigt, um Einfluss auf die genannte Transferzeit zu nehmen, nämlich eine Beeinflussung der Oberflächeneigenschaften der Kapillare 132. Aufgetragen ist hier eine Füllzeit t einer Kapillare in Sekunden als Teil der oben beschriebenen Sammelzeit, für eine Kapillare mit einer Breite von 120 Mikrometern, einer Tiefe von 80 Mikrometern und einer Länge von 8 mm als Funktion einer von Wasser zurück gelegten Distanz d in mm innerhalb der Kapillare 132. Dabei wurden Messungen an Kapillaren vorgenommen, welche auf verschiedene Weisen oberflächenbehandelt wurden. Grundsätzlich lassen sich für eine hydrophile Oberflächenbehandlung eine Vielzahl geeigneter Verfahren und/oder Beschichtungen bzw. Materialien einsetzen, die dem Fachmann grundsätzlich auch aus anderen Bereichen der Technik bekannt sind, beispielsweise Beschichtungen mit Detergenzien. Die Oberflächenbehandlung in Figur 4 umfasst dabei eine Hydrophilisierung beispielsweise durch eine geeignete hydrophile Oberflächenbeschichtung. Dabei bezeichnen in Figur 4 die Kurven mit den geschlossenen Dreieckssymbolen Messungen an Kapillaren 132 mit hydrophiler Beschichtung, wohingegen die Kurven mit den offenen Kreissymbolen Messungen an Kapillaren 132 ohne geeignete Beschichtung beschreiben.

Weiterhin geht aus der Betrachtung der Messungen in Figur 4 auch hervor, dass die Länge der Kapillare 132 sich auch auf die Füllgeschwindigkeit auswirken kann. So ist beispielsweise aus der Betrachtung der Kurven mit den geschlossenen Dreieckssymbolen ersichtlich, dass sich die Teil-Füllzeit zwischen 0 mm und 4 mm deutlich von der Teil-Füllzeit des Abschnitts zwischen 4 mm und 8 mm unterscheidet. Dementsprechend sind allgemein, unabhängig von der sonstigen Ausgestaltung des Systems, bei Systemen 110 zum Nachweis mindestens eines Analyten in einer Körperflüssigkeit, welche für einen Probentransfer mindestens eine Kapillare 132 verwenden, kurze Kapillaren 132 zu bevorzugen.

Unter Verwendung der oben beschriebenen, bevorzugten Relation zwischen zu befüllender Kapillarlänge und Kapillardurchmesser ergibt sich für offene eine Kapillare 132 mit einer Breite von 120 Mikrometern und einer Tiefe von 80 Mikrometern ein Kapillardurchmesser im Sinne der obigen Definition von 2 . 0,08 mm + 0,120 mm = 0,280 mm. Für Kapillarlängen von 8 mm ergibt sich somit ein Verhältnis Länge zu Durchmesser von 29, für Kapillarlängen von 6 mm ein Verhältnis von 21 und für Kapillarlängen von 4 mm ein Verhältnis von 14. Bevorzugt sind also im Rahmen der vorliegenden Erfindung und mit den genannten Maßen der Kapillare Kapillaren 132 mit einer Länge von nicht mehr oder sogar weniger als 8 mm, insbesondere von nicht mehr oder sogar weniger als 6 mm und besonders bevorzugt von nicht mehr oder sogar weniger als 4 mm.

Umgekehrt lassen sich aus diesen Messungen einer zurückgelegten Strecke d innerhalb einer Kapillaren 132 und den oben beschriebenen Verdunstungsraten jedoch auch Rückschlüsse ziehen auf ein Probenvolumen, welches mindestens von dem System 110 aufgenommen werden muss, um noch tolerierbare Auswirkungen der Verdunstung auf die Messergebnisse zu erhalten.

So lässt sich beispielsweise aus den in den Figuren 3 und 4 sowie in Tabelle 1 gezeigten Messergebnissen die Schlussfolgerung ziehen, dass mehr als 10 nl, vorzugsweise mindestens 12 nl, an Probenvolumen vorhanden sein müssen, wenn bei einer Verdunstungsrate von 2 nl/s und einer typischen Kapillarfüllzeit von 100 ms und einer Transferzeit von 200 ms der Fehlerbeitrag durch Verdunstung kleiner sein soll als typischerweise noch tolerierbare 5 %.

Darüber hinaus ist es, wie anhand der Figur 1 erläutert, hilfreich, Einflussgrößen, die die Verdunstung beeinflussen und die sich verändern können, gezielt durch Sensoren zu messen und bei der Auswertung der Messung zu berücksichtigen. So kann beispielsweise durch den optischen Sensor 138 ein tatsächliches Probenvolumen erfasst werden. Beispielsweise kann eine Fleckgröße einer auf ein Testfeld 129 übertragenen Probe 130 auf diese Weise erfasst werden. Auf dieses tatsächliche Probenvolumen kann dann eine Korrektur der Messung durchgeführt werden und so eine zu erwartende, relative Konzentrationsänderung durch Verdunstung berechnet beziehungsweise korrigiert werden. Beispielsweise kann mit diesem Rechenwert der gemessene Wert für die Glucosekonzentration korrigiert werden.

Für diese Korrekturen können alternativ oder zusätzlich auch weitere Parameter eingesetzt werden. Beispielsweise kann für eine derartige Korrektur zusätzlich eine Kenntnis der Temperatur, beispielsweise gemessen mittels des Sensors 142 und/oder der Luftfeuchtigkeit, beispielsweise gemessen mittels des Sensors 140, in den zur Korrektur verwendeten Rechenwert einfließen. Der Feuchtigkeitssensor 140 kann beispielsweise ein kommerziell erhältliches Hygrometer umfassen, welches Bauraum sparend ausgestaltet werden kann und kostengünstig in das System 110 implementiert werden kann.

Ein weiterer Vorschlag zur Verringerung der Einflussnahme einer Verdunstung auf die Messung der Analytkonzentration, welche alternativ oder zusätzlich zu den oben beschriebenen Möglichkeiten in ein beliebiges System 110 gemäß der vorliegenden Erfindung implementiert werden kann, kann in einer Veränderung der Geometrie der Kapillaren 132 bestehen. Beispielsweise kann ein Aspektverhältnis dieser Kapillaren verändert werden, also ein Verhältnis zwischen der Breite der Öffnung und der Tiefe des Kapillarspalts. Beispielsweise kann die Kapillare tiefer anstatt breiter ausgelegt werden, also beispielsweise 120 µm tief und lediglich 80 µm breit anstelle einer Tiefe von 80 µm und einer Breite von 120 µm. Hierdurch verringert sich bei konstantem Volumen die Oberfläche und damit die Verdunstungsrate.

### Bezugszeichenliste

- 110: System zum Nachweis eines Analyten in einer Körperflüssigkeit
- 112: Gehäuse
- 114: Innenraum
- 116: Öffnung
- 118: Finger
- 120: Lanzette
- 122: Hautpartie
- 124: Aktor
- 126: Transfervorrichtung
- 128: Testelement
- 129: Testfeld
- 130: Probe
- 132: Kapillare
- 134: Messvorrichtung
- 136: Steuerung
- 138: optischer Sensor
- 140: Feuchtigkeitssensor
- 142: Sensoren für weitere Parameter

## Patentansprüche

1. System (110) zum Nachweis mindestens eines Analyten in einer Körperflüssigkeit, wobei das System (110) eingerichtet ist, um eine Probe (130) der Körperflüssigkeit zu generieren und auf mindestens ein Testelement (128), insbesondere ein Testfeld (129), zu transferieren, wobei das System (110) derart eingerichtet ist, dass ein Probenvolumen der Probe (130) größer ist als 10 nl, wobei das Probenvolumen kleiner ist als 500 nl, wobei das System (110) eingerichtet ist, um bei der Generierung der Probe (130) und/oder dem Transfer der Probe (130) zu dem Testelement (128) eine Feuchtigkeit zu erfassen, wobei das System (110) weiterhin eingerichtet ist, um den Nachweis des Analyten unter Berücksichtigung der Feuchtigkeit vorzunehmen, wobei das System (110) mindestens eine Öffnung (116), vorzugsweise eine verschließbare Öffnung (116), aufweist, wobei eine Hautpartie, insbesondere eine Hautpartie eines Fingers, ganz oder teilweise in die Öffnung (116) einbringbar ist, wobei die Hautpartie die Öffnung (116) zumindest teilweise verschließt, wobei die Generierung der Probe (130) in der in die Öffnung (116) eingebrachten Hautpartie erfolgt.

2. System (110) nach dem vorhergehenden Anspruch, wobei das System (110) eingerichtet ist, um die Generierung der Probe (130) und den Transfer zu dem Testelement (128) innerhalb eines im wesentlichen geschlossenen Gehäuses (112) durchzuführen, wobei das System (110) eingerichtet ist, um die Feuchtigkeit innerhalb des Gehäuses (112) zu erfassen.

3. System (110) nach einem der vorhergehenden Ansprüche, wobei das System (110) eingerichtet ist, um weiterhin beim Nachweis des Analyten ein Volumen der Probe (130) zu berücksichtigen.

4. System (110) nach einem der vorhergehenden Ansprüche, wobei das System (110) weiterhin eingerichtet ist, um den Nachweis des Analyten bei Unterschreiten einer vorgegebenen Mindestfeuchtigkeit zumindest vorübergehend zu unterbrechen und/oder eine Warnung zu generieren.

5. System (110) nach einem der vorhergehenden Ansprüche, wobei das System (110) weiterhin eingerichtet ist, um bei der Generierung der Probe (130) und/oder dem Transfer der Probe (130) zu dem Testelement (128) mindestens einen weiteren Parameter zu erfassen, insbesondere innerhalb des Gehäuses (112), und vorzugsweise den mindestens einen weiteren Parameter beim Nachweis des Analyten in der Probe (130) zu berücksichtigen.

## Claims

1. A system (110) for detection of at least one analyte in a body fluid, wherein the system (110) is designed to generate a sample (130) of the body fluid and to transfer the sample to at least one test element (128), in particular a test panel (129), wherein the system (110) is designed such that a sample volume of the sample (130) is greater than 10 nl, wherein the sample volume is less than 500 nl, wherein the system (110) is designed to detect moisture during the generation of the sample (130) and/or during the transfer of the sample (130) to the test element (128), wherein the system (110) is further designed to detect the analyte taking into account the humidity, wherein the system (110) has at least one opening (116), preferably a closable opening (116), wherein a skin part, in particular a skin part of a finger, can be placed wholly or partially in the opening (116), wherein the skin part at least partially closes the opening (116), and wherein the sample (130) is generated in the skin part placed in the opening (116).

2. The system (110) according to the preceding claim, wherein the system (110) is designed to effect the generation of the sample (130) and the transfer to the test element (128) within a substantially closed housing (112), wherein the system (110) is designed to detect the humidity within the housing (112) .

3. The system (110) according to any one of the preceding claims, wherein the system (110) is designed also to take a volume of the sample (130) into account in the detection of the analyte.

4. The system (110) according to any one of the preceding claims, wherein the system (110) is further designed to at least temporarily interrupt the detection of the analyte and/or generate a warning when a predefined minimum humidity is undershot.

5. The system (110) according to any one of the preceding claims, wherein the system (110) is further designed to detect at least one further parameter, in particular within the housing (112), during the generation of the sample (130) and/or during the transfer of the sample (130) to the test element (128), and preferably to take the at least one further parameter into account in the detection of the analyte in the sample (130).

## Revendications

1. Système (110) destiné à la détection d'au moins un analyte dans un liquide corporel, le système (110) étant conçu pour produire un échantillon (130) du liquide corporel et le transférer sur au moins un élément de test (128), en particulier un champ de test (129), le système (110) étant conçu de telle sorte qu'un volume d'échantillon de l'échantillon (130) est supérieur à 10 nl, le volume d'échantillon étant inférieur à 500 nl, le système (110) étant conçu pour détecter une humidité lors de la production de l'échantillon (130) et/ou lors du transfert de l'échantillon (130) vers l'élément de test (128), le système (110) étant également conçu pour procéder à la détection de l'analyte en prenant en compte l'humidité, le système (110) présentant au moins une ouverture (116), de préférence une ouverture (116) pouvant être fermée, une partie cutanée, en particulier une partie cutanée d'un doigt, pouvant être introduite totalement ou partiellement dans l'ouverture (116), la partie cutanée fermant au moins partiellement l'ouverture (116), la production de l'échantillon (130) s'effectuant dans la partie cutanée introduite dans l'ouverture (116).

2. Système (110) selon la revendication précédente, le système (110) étant conçu pour effectuer la production de l'échantillon (130) et le transfert vers l'élément de test (128) à l'intérieur d'un boîtier (112) essentiellement fermé, le système (110) étant conçu pour détecter l'humidité à l'intérieur du boîtier (112) .

3. Système (110) selon l'une quelconque des revendications précédentes, le système (110) étant conçu pour prendre également en compte un volume de l'échantillon (130) lors de la détection de l'analyte.

4. Système (110) selon l'une quelconque des revendications précédentes, le système (110) étant également conçu pour interrompre au moins temporairement la détection de l'analyte en cas de passage au-dessous d'une humidité minimale prédéfinie et/ou pour produire un avertissement.

5. Système (110) selon l'une quelconque des revendications précédentes, le système (110) étant également conçu pour, lors de la production de l'échantillon (130) et/ou du transfert de l'échantillon (130) vers l'élément de test (128), saisir au moins un autre paramètre, en particulier à l'intérieur du boîtier (112), et pour de préférence prendre en compte l'au moins un autre paramètre lors de la détection de l'analyte dans l'échantillon (130).
